(19) Europäisches Patentamt
European Patent Office
Office européen des brevets

(11) Publication number: 0 041 488
A1

(12) # EUROPEAN PATENT APPLICATION

(21) Application number: 81850092.8

(22) Date of filing: 26.05.81

(51) Int. Cl.³: C 07 C 91/28
C 07 C 93/14, C 07 C 87/64
C 07 C 85/24, C 07 C 85/12
C 07 C 89/00, C 07 C 101/42
C 07 C 101/12, C 07 D 295/08
A 61 K 31/13

(30) Priority: 29.05.80 SE 8004002

(43) Date of publication of application:
09.12.81 Bulletin 81/49

(84) Designated Contracting States:
AT BE CH DE FR GB IT LI LU NL SE

(71) Applicant: Astra Läkemedel Aktiebolag
Strängnäsvägen 44
S-151 85 Södertälje(SE)

(72) Inventor: Arvidsson, Folke Lars-Erik
Seminariegränd 3
S-752 28 Uppsala(SE)

(72) Inventor: Carlsson, Per Arvid Emil
Torild Wulffsgatan 50
S-413 19 Göteborg(SE)

(72) Inventor: Hacksell, Uli Alf
Bandstolsvägen 4 VI
S-752 48 Uppsala(SE)

(72) Inventor: Hjorth, John Stephan Mikael
Blävalsgatan 7 A
S-414 75 Göteborg(SE)

(72) Inventor: Lindberg, Per Lennart
Knapehall 64
S-436 00 Askim(SE)

(72) Inventor: Nilsson, John Lars Gunnar
Tullinge Strand 30 B
S-146 00 Tullinge(SE)

(72) Inventor: Sanchez, Domingo
Snipäsvägen 24
S-440 03 Floda(SE)

(72) Inventor: Wikström, Häkan Vilhelm
Växelmyntsgatan 3 C
S-414 83 Göteborg(SE)

(74) Representative: Wurm, Bengt Runio et al,
Patent and Trade Mark Department Ab Astra
S-151 85 Södertälje(SE)

(54) **Therapeutically useful tetralin derivatives.**

(57) Compounds of the formula

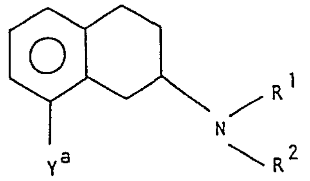

wherein $Y^a$ is $OCH_3$, OH, $NH_2$, $R^3COO$, $R^3CONH$ or $CH_3SO_2NH$ and $R^3$ is an alkyl group having 1 to 5 carbon atoms, a benzyl or phenyl group, and $R^1$ is an alkyl group having 1 to 8 carbon atoms, a benzyl or phenethyl group and $R^2$ is hydrogen or an alkyl group having 1 to 5 carbon atoms or $R^1$ and $R^2$ together form an alkylene group having 4 to 6 carbon atoms, processes for their preparation and/or pharmaceutical preparations and/or methods of treatment employing such compounds. The compounds are useful for treatment of disorders in the central nervous system.

EP 0 041 488 A1

# THERAPEUTICALLY USEFUL TETRALIN DERIVATIVES

## DESCRIPTION

### Technical Field

The present invention is related to new 1,2,3,4-tetrahydro-2-naphtylamines, to processes for preparing such compounds, pharmaceutical preparation of such compounds and the use of such compounds in therapy.

An object of the invention is to provide compounds for therapeutic use, especially compounds having a therapeutic activity in the central nervous system. A furtner object is to provide compounds having an effect on the 5-hydroxytryptamine neurones in mammals including man.

### Background Art

Compounds of the formula

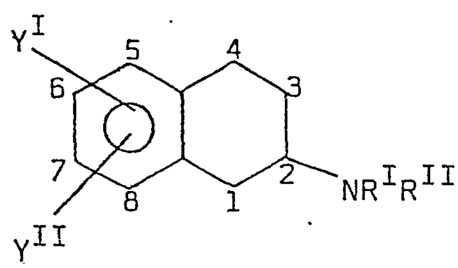

I

are disclosed by a number of references. Thus, Mc Dermed et al. (J. Med. Chem. 18, 362 (1975)) describe i.a. compounds wherein $Y^{I}$ and $Y^{II}$ are 7-OH and 8-OH, respectively, and $R^{I}$ and $R^{II}$ are both methyl or both propyl and wherein $Y^{I}$ and $Y^{II}$ are 5-OH and 8-OH, respectively, and $R^{I}$ and $R^{II}$ together are $-C_2H_4-O-C_2H_4-$. Said compounds are indicated to have dopaminergic properties although certain compounds are reported to be inactive. Further Mc Dermed et al. (J. Med. Chem. 19, 547, (1976)) describe dopaminergic compounds of formula I above, wherein $Y^{I}$ is 5-OH, 6-OH or 7-OH, $Y^{II}$ is H and $R^{I}$ and $R^{II}$ are both propyl.

BAD ORIGINAL

Rusterholz et al. (J. Med. Chem. 19, 99, (1976)) describe compounds of formula I, wherein $Y^I$ is 5-OH or 5-$OCH_3$, $Y^{II}$ is 8-OH or 8-$OCH_3$ and $R^I$ and $R^{II}$ are selected from H and methyl. Some of said compounds are potent prolactin inhibitors and believed to be dopamine agonists.

Cannon et al. (J. Med. Chem. 20, 1111, (1977)) have studied dopaminergic effects of a number of 5,6- and 6,7-dihydroxy compounds according to formula I.

Ames et al. (J. Chem. Soc. 2636 (1965)) disclose the preparation of a large number of compounds, among which are those of formula I above wherein $Y^I$ is H and $Y^{II}$ is a methoxy, ethoxy, n- or iso-propoxy, or n-, sek- or tert-butoxy group in the 5 or 8 position and $R^I$ and $R^{II}$ are H or alkyl groups having 1-4 carbon atoms. The compounds are indicated to be prepared for pharmacological testing. However, no utility or pharmacological activity is yet known for the compounds just mentioned.

Disclosure of invention

According to the present invention it has been found that novel compounds of the formula

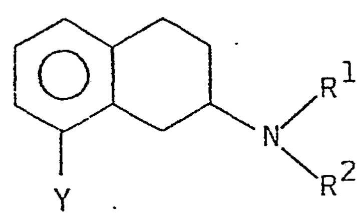

II

wherein Y is OH, $NH_2$, $R^3COO$, $R^3CONH$ or $CH_3SO_2NH$ and $R^3$ is an alkyl group having 1 to 5 carbon atoms, a benzyl or phenyl group, and $R^1$ is an alkyl group having 1 to 8 carbon atoms, a benzyl or phenethyl group and $R^2$ is hydrogen or an alkyl group having 1 to 5 carbon atoms or $R^1$ and $R^2$

together form an alkylene group having 4 to 6 carbon atoms, as well as pharmaceutically acceptable acid addition salts thereof, possess unexpected pharmacological properties rendering them useful in therapy and fulfilling the objects stated above. Processes for preparation of such compounds, their pharmaceutical and medical use and pharmaceutical preparations and methods of treatment employing such compounds constitute further aspects of the invention.

Compounds of formula IIa below wherein $Y^a$ is $OCH_3$ are intermediates in preparation of the corresponding hydroxy compounds of formula II. According to the invention said compounds are found to have valuable therapeutic properties. Thus, according to a further aspect the invention is related to the pharmaceutical and medical use of compounds as well as to pharmaceutical preparations and methods of treatment employing compounds of the formula

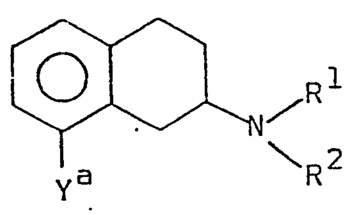

IIa

wherein $Y^a$ is $OCH_3$, OH, $NH_2$, $R^3COO$, $R^3CONH$ or $CH_3SO_2NH$ and $R^1$, $R^2$ and $R^3$ are as defined above, as well as pharmaceutically acceptable acid addition salts thereof.

Both organic and inorganic acids can be employed to form non-toxic pharmaceutically acceptable acid addition salts of the compounds of this invention. Illustrative acids are sulfuric, nitric, phosphoric, hydrochloric, citric, acetic, lactic, tartaric, pamoic, ethanedisulfonic, sulfamic, succinic, cyclohexylsulfamic, fumaric, maleic and benzoic acid. These salts are readily prepared by methods known in the art.

According to a preferred embodiment the invention is related to compounds of the formula II wherein Y is OH or $R^3COO$. Further preferred are compounds wherein $R^1$ and $R^2$ are the same or different and selected from ethyl and n-propyl. For their medical use application further compounds of formula IIa wherein $Y^a$ represents $CH_3O$ belong to the preferred group of compounds.

The compounds of formula II and IIa contain an asymmetric carbon atom in the aliphatic ring moiety, i.e. the ring carbon atom adjacent to the nitrogen atom. The therapeutic properties of the compounds may to a greater or lesser degree be ascribed to either or both of the two enantiomers occurring. Thus the pure enantiomers as well as mixtures thereof are within the scope of the invention.

The invention takes into consideration that compounds which structurally deviates from the formula II or IIa after administration to a living organism may be transformed to a compound of the formula II or IIa and in this structural form exert their effects. Likewise, certain compounds of formula II or IIa may be metabolized into other compounds of formula II before exerting their effect. Compounds of the invention wherein Y or $Y^a$ is $R^1COO$, $R^1CONH$ or $CH_3SO_2NH$ are thus believed to exert their main activity after metabolism to compounds wherein Y or $Y^a$ is OH, $NH_2$ and $NH_2$, respectively.

## Methods of Preparation

The compounds of the invention may be obtained by one of the following methods constituting a further aspect of the invention.

a) An ether of the formula

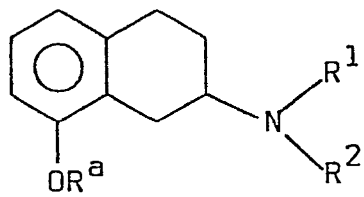

III

wherein $R^a$ represents a hydrocarbon residue, preferably an alkyl group having 1-5 carbon atoms, or a benzyl group, and $R^1$ and $R^2$ are as defined above, may be cleaved to form a compound of formula II wherein Y is a hydroxy group. The cleavage may be carried out by treating the compound of formula III with an acidic nucleophilic reagent such as aqueous HBr, or HI, $HBr/CH_3COOH$, $BBr_3$, $AlCl_3$, pyridine-HCl or $(CH_3)_3SiI$, with a basic nucleophilic reagent such as $CH_3C_6H_4-S^{\ominus}$ or $C_2H_5-S^{\ominus}$.

When $R^a$ is a benzyl group the cleavage may also be carried out by reduction, preferably with hydrogen using Pd or $PtO_2$ as catalyst.

b) A compound of formula II wherein Y is OH or $NH_2$ and $R^2$ is other than H may be converted into a compound of the same formula wherein Y is $R^1COO$, $R^1CONH$ or $CH_3SO_2NH$ by treating the firstmentioned compound with an appropriate carboxylic acid halide $R^1COX$ or anhydride $(R^1CO)_2O$ or with a methylsulfonyl halide $CH_3SO_2X$ in the presence of a base such as triethylamine or pyridine or an acid such as $H_2SO_4$ or $CF_3COOH$. X represents a halogen, preferably Cl or Br.

c) A compound of formula IV

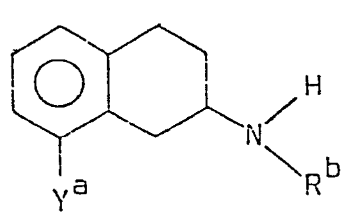

IV

wherein $R^b$ is either $R^1$ or $R^2$, and $R^1$, $R^2$ and $Y^a$ are as defined above, may be converted into a compound of formula II wherein Y is OH, by alkylation of the nitrogen atom with an appropriate alkylating agent. Thus, the starting compound wherein $R^b$ is $R^2$ may be treated with an alkyl, benzyl or phenethyl halide or tosylate $R^1X^1$, wherein $X^1$ represents Cl, Br, I or

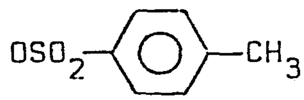

in an organic solvent such as acetonitrile or acetone and in the presence of a base such as $K_2CO_3$ or NaOH, or said starting compound may be treated with a carboxylic acid $NaBH_4$ complex $R^cCOOHNaBH_4$, wherein $R^c$ is defined by the relation $R^c$-$CH_2$- equals $R^1$. To the formation of a compound of formula I wherein at least one of $R^1$ and $R^2$ is $CH_3$, the alkylation reaction may be carried out by treatment of the compound of formula IV with a formaldehyde - $Na(CN)BH_3$ mixture, or with formaldehyde and formic acid. To the formation of a compound of formula II wherein $R^1$ is a cycloalkyl group, the alkylation may be carried out by reaction of the compound of formula IV wherein $R^b$ is $R^2$ with a cyclic ketone of the formula

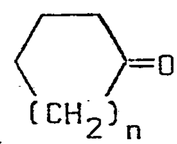

wherein n is 1, 2, or 3, whereby the intermediate of formula

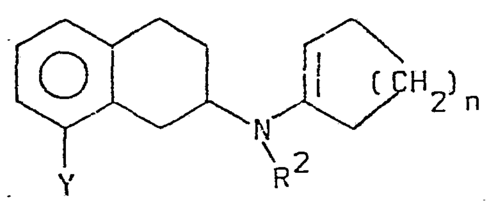

formed, is reduced, preferably with hydrogen using Pd or $PtO_2$ as catalyst.

d) An amide of the formula

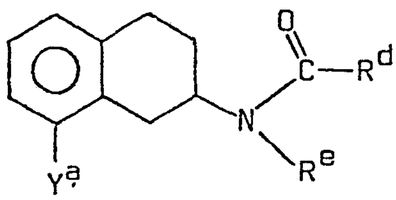

V

wherein $Y^a$ is $CH_3O$, OH or $NH_2$, $R^d$ is an alkyl group defined by the relation $R^d$-$CH_2$- equals either $R^1$ or $R^2$ and $R^e$ is the other of $R^1$ and $R^2$, may be reduced, e.g. by treatment with a hydride reducing agent such as $LiAlH_4$ in ether or tetrahydrofuran or $BH_3$ in tetrahydrofuran, to the formation of a compound of formula II or IIa.

e) A compound of the formula

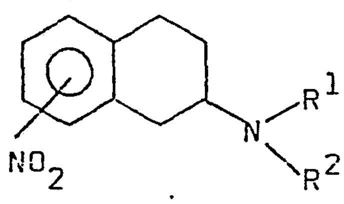

IX

may be reduced to the corresponding amine. When the starting material is an isomeric mixture as regards the position of the nitro group chromatographic separation either before or after the reduction yields the 8-amino compound of formula II. The reduction is suitably carried out with hydrogen and Pd catalyst.

f) A compound of the formula

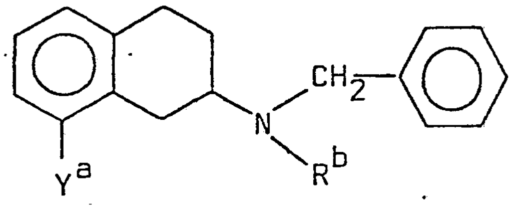

XIV

wherein $Y^a$ and $R^b$ are as defined above, may be catalytically reduced to the formation of a compound of formula IV. With appropriate meanings of $Y^a$ and $R^b$ the product obtained also satisfies formula II or IIa. Said method is especially useful in preparation of pure enantiomers as enantiomer separation of the benzyl derivatives of formula XIV may easily be done by recrystallization with optically active tartaric acid.

g) An enamine with either a $C_1$-$C_2$ or a $C_2$-$C_3$ double bond or an imine base (without $R^2$) or immonium salt (e.g. $ClO_4^-$ or $BF_4^-$) with a $C_2$-N double bond of the formula

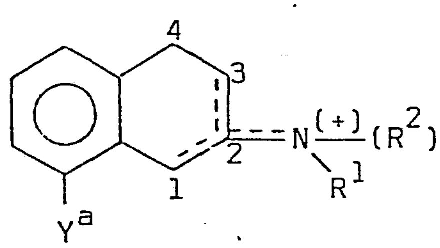

XV

wherein $Y^a$, $R^1$ and $R^2$ are as defined above may be reduced to a compound of formula II or IIa, preferably by catalytic hydrogenation using $PtO_2$ or Pd as a catalyst.

h) A compound of the formula

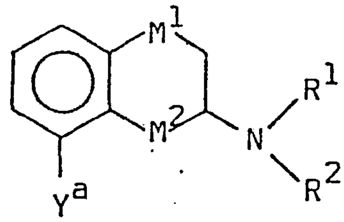

XVI

wherein $M^1$ and $M^2$ are the same or different and each represents $-CH_2-$, $>CH-Z^1$ or $>C=O$, $Z^1$ is a group sensitive to hydrogenolysis such as hydroxy in benzylic position or halogen, and $R^1$ and $R^2$ are as defined above, may be converted to a compound of formula II or IIa by reduction. Thus, a keto function may be either directly converted to $CH_2$ by treatment with e.g. hydrazine under alkaline conditions or by a stepwise reduction by using e.g. catalytic hydrogenation which may involve an intermediary formation of a hydroxy group and, where possible, also an elimination to a double bond. Reduction of a group $>CH-Z^1$ may be carried out by using a nucleophilic hydride reducing agent such as $LiAlH_4$, or catalytic hydrogenation.

i) In a compound of the formula

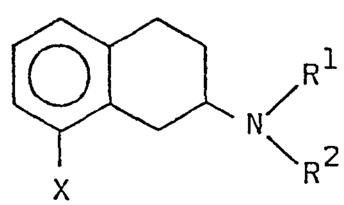

XVII

wherein X represents $SO_3H$, Cl or $NH_2$, a hydroxy group may be substituted for the group X to the formation of a compound of formula II wherein Y represents a hydroxy group. When X is $SO_3H$ or Cl said reaction may be carried out by treatment with a strong alkali under heating, suitably with an alkali melt such as KOH when X is $SO_3H$, and with a strong aqueous alkali such as NaOH or KOH when X is Cl.

When X is $NH_2$ the reaction may be carried out by treatment with aqueous nitrous acid to the formation of an intermediate diazonium compound which is then subjected to hydrolysis in water.

Free bases formed may subsequently be converted into their acid addition salts, and acid addition salts formed may subsequently be converted into the corresponding bases or other acid addition salts.

## Preparation of Starting Materials

Starting materials for the methods of preparation described above may be obtained by several methods known in the art or described below.

A) The starting material for method a) according to formula III above may be prepared by one of the following methods:

A1)

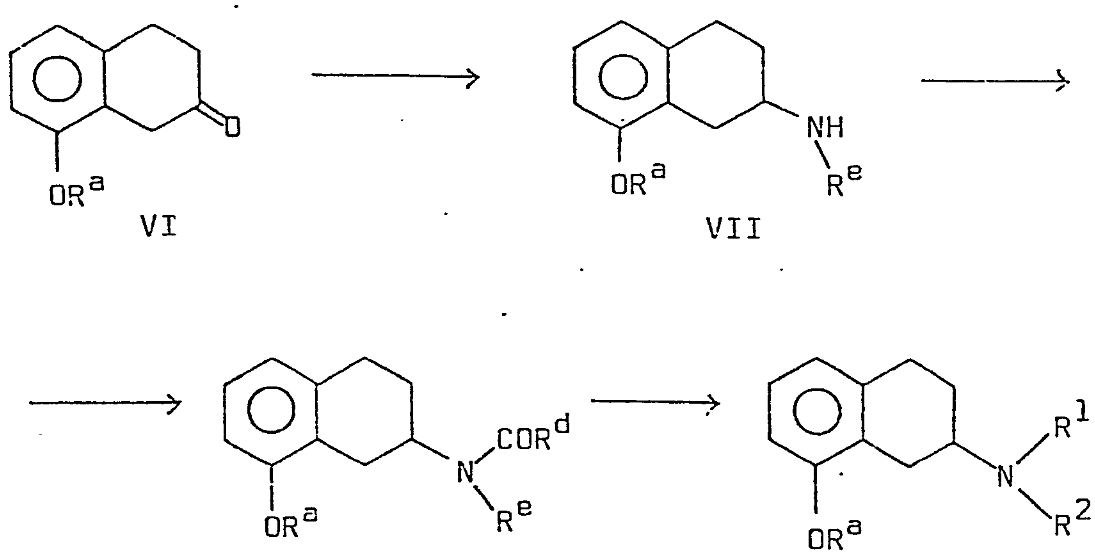

VI VII VIII III

A compound of formula VI is reacted with an amine such as $R^eNH_2$ in the presence of acetic acid and molecular sieves or methylamine hydrochloride in the presence of NaOH to the formation of an intermediate imine, which is reduced by catalytic hydrogenation to a compound of formula VII. When a tertiary amine is desired, compound VII is then acylated with a carboxylic acid chloride in the presence of triethylamine and subsequently reduced in the manner described under d) above to a compound of formula III. A compound of formula III wherein $R^2$ is H is obtainable directly from the amination of VI to VII.

BAD ORIGINAL

A2)

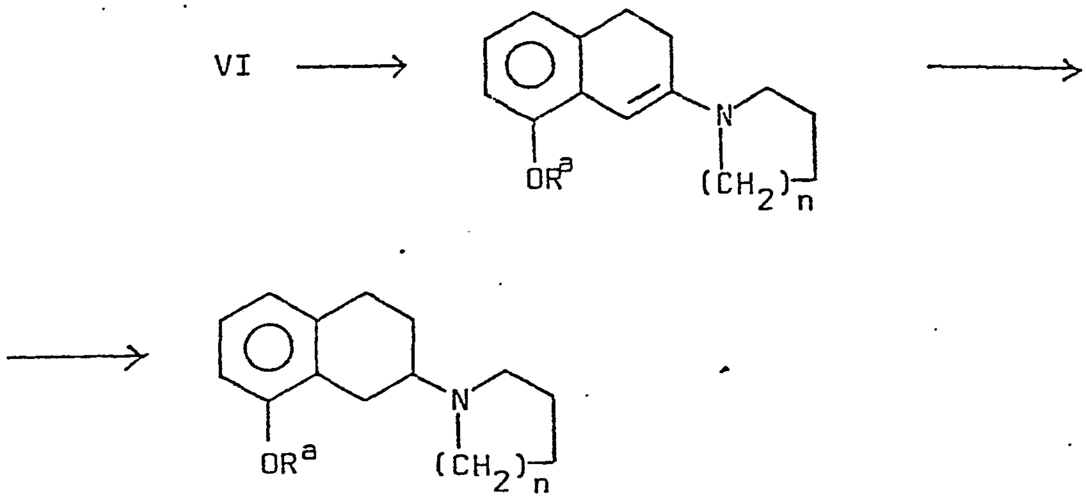


For the preparation of a starting material wherein $R^1$ is a cycloalkyl group, a compound of formula VI may be reacted with an azacycloalkane of the formula

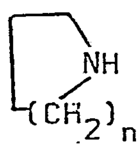

XIII

wherein n is 1, 2, or 3 and the intermediate is catalytically hydrogenated suitably with $PtO_2$ as the catalyst.

B) The starting materials for method b) are also compounds of the invention and are obtainable by a suitable method described under a), c), d), or e) above.

C) The starting materials for method c) are obtainable by a method analogous to the steps VI ⟶ VII of method A1). The hydrocarbon residue $R^a$ is suitably split off from the compound of formula VII when $Y^a$ =OH is desired in the compound of formula IV. Starting materials satisfying formula II or IIa are also obtainable by one of methods a) or d).

D) The starting materials for method d) are obtainable D1) by steps VI ⟶ VII ⟶ VIII of method A1 when in compound V $Y^a$ is $CH_3O$. When $Y^a$ is OH the starting compound is obtainable D2) by splitting off the hydrocarbon residue $R^a$ in compound VIII, suitably with $BBr_3$. When $Y^a$ is $NH_2$ the starting compound is obtainable D3) by reduction of a compound of the formula

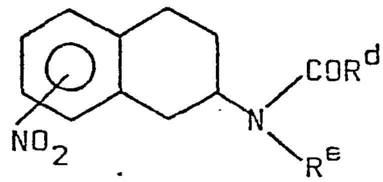

X

in a manner analogous to method e) involving chromatographic separation. The compound X is obtainable by the reactions

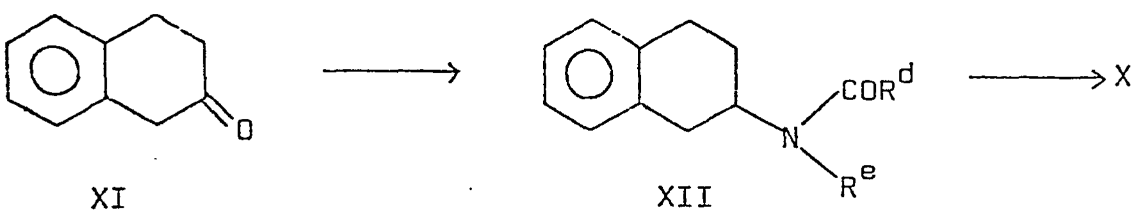

XI XII

The compound XII is prepared from XI in analogy with steps VI ⟶ VII ⟶ VIII of method A1). Compound X is then obtained by treatment with $HNO_3$, $H_2SO_4$ and water.

E) Starting materials for method e) are obtainable by amination of a compound of formula XI. The amination may be carried out in analogy with method A1 or A2 above. In the intermediate of formula

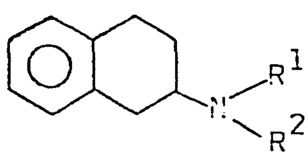

formed a nitro group is then introduced in analogy with the reaction XII ⟶ X according to D3 above.

BAD ORIGINAL

F) Starting materials of formula XIV employed according to method f) are obtainable by one of the above methods.

Pharmaceutical preparations

Pharmaceutical preparations of the compounds of the invention constitute a further aspect of the invention.

In clinical practice the compounds of the present invention will normally be administered orally, rectally, or by injection, in the form of pharmaceutical preparations comprising the active ingredient either as a free base or as a pharmaceutically acceptable non-toxic, acid addition salt, e.g. the hydrochloride, lactate, acetate, sulfamate, and the like, in association with a pharmaceutically acceptable carrier.

Accordingly, terms relating to the novel compounds of this invention, whether generically or specifically, are intended to include both the free amine base and the acid addition salts of the free base, unless the context in which such terms are used, e.g. in the specific examples, would be inconsistent with the broad concept. The carrier may be a solid, semisolid or liquid diluent or capsule. These pharmaceutical preparations constitute a further aspect of this invention. Usually the active substance will constitute between 0.1 and 99% by weight of the preparation, more specifically between 0.5 and 20% by weight for preparations intended for injection and between 0.2 and 50% by weight for preparations suitable for oral administration.

Pharmaceutical preparations containing a compound of the invention may preferably contain between 2 and 50% by weight of the active substance, in such preparations the selected compound may be mixed with a solid fine grain carrier, e.g. lactose, saccharose, sorbitol, mannitol, starches such as potato starch, corn starch or amylopectin, cellulose derivatives, or gelatin and a lubricant such as magnesium

stearate, calcium stearate, polyethylene glycol waxes, and the like, and then compressed to form tablets. If coated tablets are required, the cores, prepared as described above, may be coated with a concentrated sugar solution which may contain, e.g. gum arabic, gelatin, talcum, titanium dioxide, and the like. Alternatively the tablet can be coated with a lacquer dissolved in a readily volatile organic solvent or mixture of organic solvents. Dyestuffs may be added to these coatings in order to readily distinguish between tablets containing different active substances or different amounts of the active compound.

For the preparation of soft gelatin capsules (pearl-shaped closed capsules) consisting of gelatin and, for example, glycerol, or similar closed capsules, the active substance may be admixed with a vegetable oil. Hard gelatin capsules may contain granulates of the active substance in combination with solid, fine grain carriers such as lactose, saccharose, sorbitol, mannitol, starches (e.g. potato starch, corn starch or amylopectin), cellulose derivatives or gelatin.

Liquid preparations for oral application may be in the form of syrups or suspensions, for example, solutions containing from about 0.2% to about 20% by weight of the active substance herein described, the balance being sugar and a mixture of ethanol, water, glycerol and propyleneglycol. Optionally such liquid preparations may contain colouring agents, flavouring agents, saccharine and carboxymethylcellulose as a thickening agent.

Solutions for parenteral applications by injection can be prepared in an aqueous solution of a water-soluble pharmaceutically acceptable salt of the active substance preferably in a concentration of from about 0.5% to about 10% by weight. These solutions may also contain stabilizing agents and/or buffering agents and may conveniently be provided in various dosage unit ampoules.

In therapeutical treatment the suitable daily doses of the compounds of the invention are 1-2000 mg for oral application, preferentially 50-500 mg, and 0.1-100 mg for parenteral application, preferentially 0.5-50 mg.

Working examples

The following examples will further illustrate the invention.

Preparation of intermediates and compounds of formula IIa wherein $Y^a$ is $CH_3O$

Example I1: 2-(n-propylamino)-8-methoxytetralin (Method A1)

To a solution of 8-methoxy-2-tetralone (10.2 g, 58 mmol) in absolute ethanol (175 ml) over 4 Å molecular sieves (19.0 g) were added n-propylamine (10.3 g, 174 mmol) and acetic acid (10.4 g, 174 mmol). The mixture was shaken in a closed flask ($N_2$ atmosphere) for half an hour, heated to 70°C and allowed to cool. The molecular sieves were filtered off, and the solution was hydrogenated catalytically over $PtO_2$ at atmospheric pressure. After filtration and evaporation, the acetate of the title compound was washed with ether, and made alkaline with saturated $Na_2CO_3$ solution. The aqueous phase was extracted with ether and the combined organic layers were evaporated. The residue was passed through an alumina column with ether as eluant, precipitated as the hydrochloride and recrystallized from ethanol/ether.

Example I2. 2-(di-n-propylamino)-8-methoxytetralin (Method A1)

Propionylchloride (5.4 g, 58 mmol) in dry toluene (10 ml) was slowly added at 5°C to a solution of 2-(propylamino)-8-methoxytetralin (8.5 g, 39 mmol), triethylamine (5.9 g, 58 mmol) and dry toluene (60 ml). The mixture was stirred at room temperature for 30 min, whereupon the triethylammonium chloride formed was filtered off and the solvent evaporated. The residue was passed through an alumina column with ether/light petroleum as eluant. After evaporation the amide dissolved in dry tetrahydrofuran (30 ml) was added to a suspension of $LiAlH_4$ (2.0 g, 53 mmol) in dry tetrahydrofuran (100 ml) under nitrogen. After stirring under reflux for 5 h, the mixture was hydrolyzed, the precipitate was filtered off, and the solvent was evaporated. The residue was passed through an alumina column with ether//light petroleum (1+2), and the title compound was precipitated as the hydrochloride and recrystallized from ethanol/ether.

Example I3. 2-piperidino-8-methoxytetralin
(Method A2)

A solution of 8-methoxy-2-tetralone (10.0 g, 57 mmol), piperidine (14.5 g, 170 mmol), and p-toluenesulfonic acid (0.5 g) in dry toluene (500 ml) was refluxed for 24 h. The solvent was evaporated and the residue was dissolved in absolute ethanol and transferred to a Parr flask. $PtO_2$ (0.5 g) was added and the hydrogenation was performed at 0.38 MPa gauge. The catalyst was filtered off, the solvent was evaporated, and the residue was passed through an alumina column with ether/light petroleum (1+1) as eluant. The title compound was precipitated as the hydrochloride and recrystallized from ethanol.

Example I4. 2-(methylamino)-8-methoxytetralin
(Method A1)

8-methoxy-2-tetralone (5.0 g, 28 mmol) was dissolved in ethanol under nitrogen. Methylamine hydrochloride (3.8 g, 56 mmol) dissolved in water (50 ml) and NaOH (5.6 g, 140 mmol), were added under stirring. After stirring for 20 min, the mixture was poured onto ice (100 g) mixed with concentrated HCl (30 ml). The acidic solution was hydrogenated catalytic-

ally over $PtO_2$ (150 mg) in a Parr apparatus at 0.34 MPa gauge. After filtration, the ethanol was evaporated, and the remaining aqueous phase was poured into water (100 ml), washed with ether and made alkaline. Extraction with ether and evaporation gave the crude solid amine, which was passed through an alumina column with ether as eluant. The title compound was precipitated as the hydrochloride and recrystallized from i-propanol/ether.

Example I5. 8-amino-2-(N-n-propyl-N-propionyl)amino-tetralin (Method D3)

2-(N-n-propyl-N-propionyl)aminotetralin (59.9 g, 0.24 mol) (prepared according to A1 from 2-tetralone in 86% yield) was dissolved in $CH_3NO_2$ (1000 ml). A mixture of concentrated $HNO_3$ (16.5 ml), concentrated $H_2SO_4$ (218 ml) and water (35.4 ml) was added dropwise under cooling and thorough stirring maintaining the temperature between 0-10$^oC$. The mixture was stirred at 0$^oC$ for another 2 hours and then poured onto ice-water (4 l). Extraction with $CH_2Cl_2$, washing the organic phase with water, drying ($Na_2SO_4$) and evaporation of the solvent gave a crude oil (64.4 g) of an isomeric mixture of 5-, 6-, 7- and 8-mononitro-2-(N-n-propyl-N-propionyl)aminotetralin. The mixture was dissolved in ethanol (1200 ml). Acetic acid (50 ml) and 10% Pd/C (3 g) was added and the mixture was hydrogenated at 0.34 MPa gauge. The catalyst was filtered off and the solvent evaporated giving a residual oil. The oil was dissolved in 1 M HCl (500 ml), and the solution was extracted with ether. The ether phase was discarded, the aqueous phase was made alkaline ($Na_2CO_3$) and extracted with ether. The organic phase was dried ($Na_2SO_4$) and the solvent evaporated giving a crude oil (57 g) of an isomeric mixture of 5-, 6-, 7- and 8-monoamino--2-(N-n-propyl-N-propionyl)aminotetralin.

A small amount (1.0 g) of the last-mentioned isomeric mixture was converted to the hydrochloride and chromatographed on an RP-18 column using MeOH-2 M NaOAc (1:1) + 2 % HOAc as eluant. (In this way all the isomers could be separated). The last fraction (a total volume of about 1000 ml), containing the pure 8-isomer (HPLC), was worked up by first evaporating off the methanol, making the residual solution alkaline with 20 % NaOH and extracting with ether. The ether phase was dried ($Na_2SO_4$) and the solvent evaporated giving a residue (80 mg) of the desired product 8-amino-2-(N-n-propyl-N-propionyl)aminotetralin (NMR).

Example I6. (+)-2-benzylamino-8-methoxytetralin

(+)-Tartaric acid (15.92 g, 0.106 mol) dissolved in hot ethanol (500 ml) was added to a hot solution of (±)-2--benzylamino-8-methoxytetralin (28.36 g, 0.106 mol) in ethanol (1500 ml). The solution was allowed to stand over night at room temperature. The crystals thus formed was recrystallized four times by dissolving in boiling ethanol and cooling to room temperature. The resolved amine was released with NaOH solution into ether. The organic layer was dried ($K_2CO_3$) and the solvent was removed under reduced pressure yielding (+)-2-benzylamino-8-methoxytetralin. A small sample was converted to the hydrochloride for analysis, melting point and determination of specific rotation.

The enantiomeric purity of the amine was determined by analysis of the NMR-spectrum of its O-methylmandelamide which was prepared as follows: R(-)-2-Methoxy-2-phenylacetylchloride (155 mg, 0.8 mmol) dissolved in dry benzene (5 ml) was added to an ice-cooled solution of (+)-2-benzylamino--8-methoxytetralin (150 mg, 0.6 mmol) and triethylamine (170 mg, 1.7 mmol) in dry benzene (20 ml). The mixture was

stirred at room temperature for 1 hour whereupon it was filtered and the solvent evaporated. The residue, dissolved in ether, was washed with 2N HCl, 2N NaOH and water. After drying ($K_2CO_3$) the organic layer the ether was distilled off and the crude amide was dissolved in dimethylsulfoxide-$d_6$. In the nmr-spectrum of the diastereomeric amides the methin proton alpha to the methoxygroup gave singlets with different chemical shifts (5.36 and 5.44 ppm). The enantiomeric purity was determined to more than 93 % by estimation of the relative areas under these peaks.

Example I7. (+)-2-propylamino-8-methoxytetralin (Method F)

The hydrochloride of (+)-2-(N-n-benzyl-N-n-propylamino)--8-methoxytetralin, prepared from (+)-2-benzylamino-8-methoxytetralin (3.65 g, 13.65 mmol) according to method A1, d,(Example I2), was dissolved in methanol (100 ml): Pd on charcoal catalyst was added and hydrogenolysis was performed at atmospheric pressure. Removal of catalyst and solvent gave (+)-2-propylamino-8-methoxytetralin as the hydrochloride, which was treated with 2N NaOH. The free amine was extracted with ether. The ether layer was removed, dried ($K_2CO_3$) and evaporated. The amine was purified on an alumina column with ether as eluant and converted to its hydrochloride and recrystallized from ethanol/ether.

Preparation of end compounds of formula II

Example E1. 2-(di-n-propylamino)-8-hydroxytetralin hydrobromide (Method a)

2-(di-n-propylamino)-8-methoxytetralin hydrochloride (4.5 g were suspended in 48 % HBr (100 ml). The mixture was then heated at 120°C for 2 h under nitrogen. The hydrobromic

acid was evaporated, water was added and evaporated. The residue was recrystallized from ethanol yielding the pure 2-(di-n-propylamino)-8-hydroxytetralin hydrobromide.

Example E2. 8-amino-2-di-n-propylaminotetralin hydrochloride (Method d)

8-amino-2-(N-n-propyl-N-propionyl)aminotetralin (80 mg, 0.31 mmol) dissolved in dry ether (10 ml) was added dropwise to a stirred mixture of $LiAlH_4$ (0.2 g) in dry ether (10 ml) ($N_2$ atmosphere) and then the mixture was stirred for 30 min at room temperature. Addition of water (0.2 ml), 15 % NaOH (0.2 ml) and finally water (0.6 ml) followed by filtration, drying ($Na_2SO_4$) of the filtrate and evaporation of the ether gave the product base as a residual oil (90 mg) (NMR).

The base was converted to the hydrochloride by dissolving in ether and precipitating with HCl-saturated ether. The hydrochloride was recrystallized from ethanol-ether.

According to the methods of the Examples above the following compounds were prepared and recrystallized as acid addition salts from ethanol/ether or isolated as the bases.

Compounds and experimental data

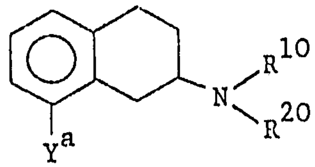

| Compound/ Example No. | $Y^a$ | $R^{10}$ | $R^{20}$ | Salt | Method (Ref. to Example No.) | Melting point (°C) or other data a) | $[\alpha]_D^{22}$ c) | Yield(%) |
|---|---|---|---|---|---|---|---|---|
| 1/I4 | $CH_3O$ | $CH_3$ | H | HCl | A1 | 140-141 | | 50 |
| 2/I1 | $CH_3O$ | $n-C_3H_7$ | H | HCl | A1, | 193-194 | | 78 |
| (+)-2/I7 | $CH_3O$ | $n-C_3H_7$ | H | HCl | f | 236-237 | +78.3° (c 1.05) | 80 d) |
| (-)-2 | $CH_3O$ | $n-C_3H_7$ | H | HCl | f | 235-236.5 | -76.5° (c 1.03) | 78 d) |
| 3 | $CH_3O$ | $n-C_4H_9$ | H | HCl | A1 | 191.5-193.0 | | 55 |
| 4 | $CH_3O$ | $CH(CH_3)_2$ | H | HCl | A1 | 248-248.5 | | 55 |
| 5 | $CH_3O$ | $n-C_8H_{17}$ | H | HCl | A1 | 164-165 | | 43 |
| 6 | $CH_3O$ | $CH_2$- 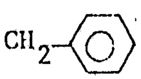 | H | HCl | A1 | 218.5-219.5 | | 62 |
| (+)-6/I6 | $CH_3O$ | $CH_2$- 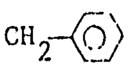 | H | HCl | | 239-240 | +63.3° (c 1.01 | 23 d) |
| (-)-6 | $CH_3O$ | $CH_2$- 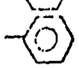 | H | HCl | (I6) | 240-241 | -62.5° (c 1.01) | 12 d) |
| 7 | $CH_3O$ | $C_2H_5$ | $n-C_3H_7$ | HCl | A1, d | 131.5-132.5 | | 94 e) |
| 8/I2 | $CH_3O$ | $n-C_3H_7$ | $n-C_3H_7$ | HCl | A1, d | 148.5-149.5 | | 85 |
| (+)-8 | $CH_3O$ | $n-C_3H_7$ | $n-C_3H_7$ | HCl | A1, d | 164-165 | +77.1 (c 1.04) | 88 d) |
| (-)-8 | $CH_3O$ | $n-C_3H_7$ | $n-C_3H_7$ | HCl | A1, d | 164-164.5 | -76.1 (c 1.00) | 85 d) |
| 9 | $CH_3O$ | $n-C_3H_7$ | $n-C_4H_9$ | HCl | A1, d | 136-138 | | 94 e) |

Compounds and experimental data (cont.)

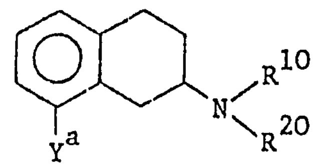

| Compound/ Example No. | $Y^a$ | $R^{10}$ | $R^{20}$ | Salt | Method (Ref. to Example No.) | Melting point ($^oC$) or other data[a)] | $[\alpha]_D^{22}$ [c)] | Yield (%) |
|---|---|---|---|---|---|---|---|---|
| 10 | $CH_3O$ | $n-C_4H_9$ | $n-C_4H_9$ | HCl | A1, d | 135-136.5 | | 91 |
| 11 | $CH_3O$ | $n-C_3H_7$ | $-CH_2-$ 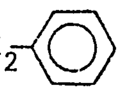 | HCl | A1, d | 192-192.5 | | 95[f)] |
| 12/I3 | $CH_3O$ | $-(CH_2)_5-$ | | HCl | A2 | 281.5-282.5 | | 61 |
| 13/I5 | $NH_2$ | $n-C_3H_7$ | $COC_2H_5$ | - | D3 | NMR[b)] | | |
| 14 | HO | $CH_3$ | H | HBr | a | 220-220.5 | | 70 |
| 15 | HO | $n-C_3H_7$ | H | HBr | a | 245-247 | | 60 |
| 16 | HO | $n-C_4H_9$ | H | HBr | a | 195-196 | | 26 |
| 17 | HO | $CH(CH_3)_2$ | H | HBr | a | 222-223 | | 82 |
| 18 | HO | $n-C_8H_{17}$ | H | HBr | a | 213-214.5 | | 46 |
| 19 | HO | $C_2H_5$ | $n-C_3H_7$ | HBr | a | 176.5-177.5 | | 45 |
| 20/E1 | HO | $n-C_3H_7$ | $n-C_3H_7$ | HBr | a | 221.5-222.5 | | 89 |
| (+)-20 | HO | $n-C_3H_7$ | $n-C_3H_7$ | HBr | a | 178.5-179.5 | $+67.5^o$ (c 1.03) | 89[d)] |

Compounds and experimental data (cont.)

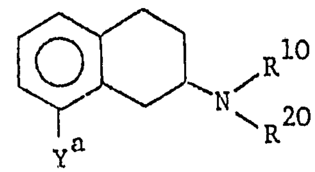

| Compound/ Example No. | $Y^a$ | $R^{10}$ | $R^{20}$ | Salt | Method (Ref. to Example No.) | Melting point (°C) or other data[a)] | $[\alpha]_D^{22}$ [c)] | Yield (%) |
|---|---|---|---|---|---|---|---|---|
| (-)-20 | HO | n-$C_3H_7$ | n-$C_3H_7$ | HBr | a | 178.5-179.5 | -67.5° (c 1.01) | 82[d)] |
| 21 | HO | n-$C_3H_7$ | n-$C_4H_9$ | HBr | a | 175.5-177 | | 42 |
| 22 | HO | n-$C_4H_9$ | n-$C_4H_9$ | HBr | a | 176-178 | | 52 |
| 23 | HO | n-$C_3H_7$ | $CH_2$- 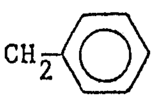 | HBr | a | 216.5-217.5 | | 76 |
| 24 | HO | $-(CH_2)_5-$ | | HBr | a | 255.5-256.5 | | 69 |
| 25/E2 | $NH_2$ | n-$C_3H_7$ | n-$C_3H_7$ | HCl | d | 249 | | |
| 26 | 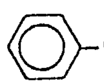 -COO- | n-$C_3H_7$ | n-$C_3H_7$ | HCl | b | 143.5-145 | | 85 |
| 27 | $CH_3O$ | $C_2H_5$ | H | HCl | A1 | 238-239 | | 51 |
| 28 | $CH_3O$ | $C_2H_5$ | $C_2H_5$ | HCl | A1, d | 148.5-149.5 | | 72 |
| 29 | $CH_3COO$ | n-$C_3H_7$ | n-$C_3H_7$ | | b | NMR[g)] | | 74 |

Footnotes to the preceding table

a) All salts, except for compound 25, were submitted for elemental analysis (C, H, N); All the analyses were satisfactory.

b) NMR $\delta(CDCl_3)$ 0.9(3H, t), 1.15(3H, t), 1.4-3.7(15H, m), 6.35-6.7(2H, m), 6.95(1H, t)

c) Measured in methanol, enantiomeric purity >93%.

d) Yield from theoretical amount of isomer in racemate.

e) Compound 2 as starting material.

f) Compound 6 as starting material.

g) NMR $\delta(CDCl_3)$ 0.89 (6H, t), 1.44 (4H, m), 1.2-2.2 (2H, m), 2.27 (3H, s), 2.47 (4H, t), 2.3-3.1 (5H, m), 6.7-7.26 (3H, m).

The following examples illustrate how the compounds of formula II or IIa employed according to the present invention may be included into pharmaceutical preparations.

Example P1. Preparation of soft gelatine capsules

500 g of active substance are mixed with 500 g of corn oil, whereupon the mixture is filled in soft gelatine capsules, each capsule containing 100 mg of the mixture (i.e. 50 mg of active substance).

Example P2. Preparation of tablets

0.5 kg of active substance are mixed with 0.2 kg of silicic acid of the trade mark Aerosil. 0.45 kg of potato starch and 0.5 kg of lactose are mixed therewith and the mixture is moistened with a starch paste prepared from 50 g of potato starch and distilled water, whereupon the mixture is granulated through a sieve. The granulate is dried and sieved, whereupon 20 g of magnesium stearate are mixed into it. Finally the mixture is pressed into tablets each weighing 172 mg.

Example P3. Preparation of a syrup

100 g of active substance are dissolved in 300 g of 95 % ethanol, whereupon 300 g of glycerol, aroma and colouring agents (q.s.) and 1000 ml of water are mixed therein. A syrup is obtained.

Example P4. Preparation of an injection solution

Active substance (hydrobromide) (1 g), sodiumchloride (0.8 g) and ascorbic acid (0.1 g) are dissolved in sufficient

amount of distilled water to give 100 ml of solution. This solution, which contains 10 mg of active substance per ml, is used in filling ampoules, which are sterilized by heating at 120$^{\circ}$C for 20 minutes.

Pharmacology

Pharmacological treatment of depression in man

Evidence is available that in depressed patients the neurotransmission in the central nervous system (CNS) may be disturbed. These disturbances appear to involve the neurotransmitters noradrenaline (NA) and 5-hydroxytryptamine (5-HT). The drugs most frequently used in the treatment of depression are considered to act by improving the neurotransmission of either or both of these phsyiological agonists. Available data suggest that the enhancement of 5-HT neurotransmission will primarily improve the depressed mood and anxiety, whereas the enhancement of noradrenaline neurotransmission will rather improve the retardation symptoms occurring in depressed patients. In recent years many efforts have been made to develop new drugs with high selectivity for the improvement of the 5-HT neurotransmission in the CNS.

The mechanism of action for the drugs generally used today in the therapy of mental depression is indirect, i.e. they act by blocking the reuptake of the neurotransmitters (NA and/or 5-HT) released from nerve terminals in the CNS, thus increasing the concentration of these transmitters in the synaptic cleft and hence restoring an adequate neurotransmission. The new antidepressive agent (Z)-Dimethylamino-1-(4-bromophenyl)-1-(3-pyridyl)propene (zimelidine) with documented good clinical effects is believed to act as such a reuptake inhibitor with high selectivity for 5-HT neurons.

A fundamentally different way to improve the neurotransmission in the central 5-HT-neurons would be to use a direct 5-HT-receptor agonist. In order to minimize side effects, a high selectivity for this kind of receptors would then be preferable.

While searching for new dopamine (DA)-receptor agonists, we surprisingly found that a group of compounds of the formula II and IIa instead had a selective, direct stimulating effect on central 5-HT receptors.

In order to evaluate the 5-HT-receptor stimulating effect and selectivity, the compounds were tested both behavioural and biochemically.

Behavioural and biochemical activity in reserpine-pretreated rats.

a. Antagonism of the reserpine-induced "neuroleptic syndrome" in the rat (gross behaviour).

Depletion of the monoamine stores with reserpine brings about a "neuroleptic syndrome" characterized by hypomotility, catalepsy, muscle rigidity, hunch-backed posture as well as a number of other central and peripheral signs of monoamine depletion. The whole or parts of this syndrome can be reversed by the administration of drugs that stimulate DA or 5-HT receptors directly or indirectly.

Stimulation of the DA receptors (e.g. with apomorphine) gives rise to both locomotion and stereotyped behaviour such as sniffing, gnawing and jumping. On the other hand, stimulation of the 5-HT receptors [e.g. with 5-hydroxytryptophan (5-HTP) combined with MAO-inhibitors], gives rise to a very different behaviour. The animals lie flat on the cage floor exhibiting forward movements with extended

BAD ORIGINAL

forepaws padding ("piano-playing") and abducted hindlegs, occasionally with some tremor in the forebody and with Straubtail(stiff tail i.a.) reaction.

b. In-vivo determination of rat brain tyrosine and tryptophan hydroxylation after reserpine pretreatment (biochemical DA- and 5-HT receptor activity).

The compounds under evaluation were tested biochemically for central DA- and 5-HT-receptor (pre- and/or postsynaptic) stimulating activity. The concept of this biochemical screening method is that a DA- or 5-HT-receptor agonist will stimulate the receptor and through regulatory feedback systems effect a decline in tyrosine or tryptophan hydroxylating activity, respectively, and a subsequent reduction in the synthesis rate for DA and 5-HT in the presynaptic neuron. Dopa and 5-HTP formation. as determined after in-vivo inhibition of the aromatic L-amino acid decarboxylase with NSD 1015 (3-hydroxybenzylhydrazine hydrochloride) are taken as indirect measures of DA- and 5-HT-synthesis rates, respectively.

Analogous conditions probably exist also for central NA-neurons. Effects on the dopa formation in the NA-predominated hemispheral parts (mainly cortex) may thus be considered to reflect NA-receptor-mediated changes.

c. Experimental procedures.

Rats (150-300 g) pretreated with reserpine (5 mg/kg, 18 h before) were given the compounds under evaluation. Gross behavioural observations (changes in motility, hindleg abduction etc; c.f. above) were made. Subsequent administration of NSD 1015, decapitation, brain dissection [corpora striata, the limbic forebrain and the remaining hemispheral portions (mainly cortex) of rat brain], homogenization,

centrifugation, ion-exchange chromatography and spectrofluorimetric measurements (all as described in detail by Wikström et al., J. Med. Chem., 21, 864-867, 1978 and references cited therein) gave the actual dopa and 5-HTP levels. Several doses (n=4-6) were tested for each compound and brain area. The dose of a compound giving 70 % of the control 5-HTP level in the rat brain part was then estimated. These values ("$ED_{70\%}$") are presented in Table 1.

d. Results.

All the compounds in Table 1 were both behaviourally and biochemically active, producing the above mentioned effects indicating central 5-HT-receptor stimulation. The absence of significant decreases in the dopa levels in the different brain parts suggests that none of the compounds possess central NA- or DA-receptor stimulating effects at the dosage under consideration.

The investigated compounds may be classified into 3 subgroups with regard to biochemical potency: 1) those with an "$ED_{70\%}$" between 0.01-1.0 µmol/kg such as compound 20 and compound 21 which are considered to be most advantageous for medical use, 2) those with an "$ED_{70\%}$" between 1.0-10 µmol/kg such as compounds 17 and 23 and 3) those with an "$ED_{70\%}$" above 10 µmol/kg such as compounds 18 and 10 which are considered to be of borderline interest. The resolved isomers were found to be about as potent as the corresponding racemates.

TABLE 1

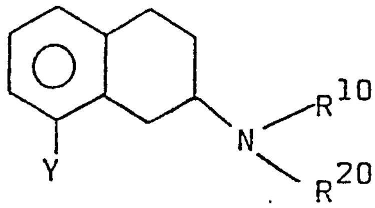

| Compound No. | Y | $R^{10}$ | $R^{20}$ | Salt | "$ED_{70\%}$"(μmol/kg, s.c.) for 5-HTP reduction a),b),c) |
|---|---|---|---|---|---|
| 8 | $CH_3O$ | $n-C_3H_7$ | $n-C_3H_7$ | HCl | 0.4 |
| (+)-8 | $CH_3O$ | $n-C_3H_7$ | $n-C_3H_7$ | HCl | 0.2-0.4 |
| (-)-8 | $CH_3O$ | $n-C_3H_7$ | $n-C_3H_7$ | HCl | 0.3-0.4 |
| 10 | $CH_3O$ | $n-C_4H_9$ | $n-C_4H_9$ | HCl | 25 |
| 14 | HO | $CH_3$ | H | HBr | 1.5-10 |
| 15 | HO | $n-C_3H_7$ | H | HBr | 0.4 |
| 16 | HO | $n-C_4H_9$ | H | HBr | 6 |
| 17 | HO | $CH(CH_3)_2$ | H | HBr | 8 |
| 18 | HO | $n-C_8H_{17}$ | H | HBr | 50 |
| 19 | HO | $C_2H_5$ | $n-C_3H_7$ | HBr | 0.09 |
| 20 | HO | $n-C_3H_7$ | $n-C_3H_7$ | HBr | 0.06 |
| (+)-20 | HO | $n-C_3H_7$ | $n-C_3H_7$ | HBr | |
| (-)-20 | HO | $n-C_3H_7$ | $n-C_3H_7$ | HBr | |
| 21 | HO | $n-C_3H_7$ | $n-C_4H_9$ | HBr | 0.5 |
| 22 | HO | $n-C_4H_9$ | $n-C_4H_9$ | HBr | 8 |
| 23 | HO | $n-C_3H_7$ | $CH_2$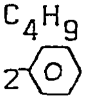 | HBr | 3 |
| 24 | HO | $-(CH_2)_5$ | - | HBr | <20 |

a) "$ED_{70\%}$" represents the dose giving 70 % of the control 5-HTP level.

b) Similar values were obtained for all the brain parts [the limbic forebrain, striatum and the hemispheral portions (mainly cortex)] investigated.

c) None of the compounds tested gave any significant reduction in dopa formation in the brain parts investigated at doses 8 times the "$ED_{70\%}$".

Effect on the sexual functions

Due to the strong and selective 5-HT-stimulating effect of the compounds of the invention, a few of these were also tested on the hetero-sexual behaviour in male rats. There is considerable evidence that central 5-HT receptor activation is involved in mediating the sexual behaviour.

During these pharmacological investigations it was revealed (see below) that the compounds of the invention have a very strong activity on the sexual behaviour in male rats. In the masculine mating pattern a marked (dose dependent) decrease was found in the number of intromissions as well as mounts preceding ejaculation, and in the ejaculatory latency, while the period of sexual inactivity following ejaculation was not significantly changed. The efficacy (maximal effect) in this respect for the compounds tested seems to be higher than for any other compound known (and tested).

It was (in preliminary experiments) further found that male rats rendered sexual inactive by castration became sexual active after treatment with compound 8.

Due to these qualities of the compounds of the invention, also a possible therapeutic application in the treatment of sexual disturbances may certainly be foreseen.

Experimental procedure

Male Wistar rats (12 months old; sexually experienced) were injected i.p. every second day in a balanced order with different doses of the compounds dissolved in physiological saline and injected in a volume of 2 ml/kg. Testing was begun 15 min after the drug treatment.

Mating arenas were 50 cm diameter acrylic glass cages with wood shavings on the floor. The stimulus females were of Wistar strain, primed with estradiol benzoate (20 μg/animal) 48 h prior to testing and progesterone (0.5 ml/animal) 4-6 h before testing.

The occurrence of mounts, intromissions and ejaculations were scored by an experienced observer. The measures of masculine copulatory behavior further included mount latency and intromission latency which are time from introduction of the stimulus female to the first mount and first intromission respectively. A copulatory series includes the mounts and intromissions preceding an ejaculation. Ejaculatory latency is the time from the first intromission in a copulatory series to the ejaculation terminating that series. The postejaculatory interval is the time from an ejaculation to the next intromission. Tests were terminated when the rat had achieved one ejaculation and started the second series of copulations, or, if the intromission latency was >15 min, the ejaculation latency was >30 min, or the postejaculatory interval was >15 min.

Results

a. Normal rats.

Results obtained for racemic 2-dipropylamino-8-methoxytetralin (compound 8), its resolved enantiomers (compounds (+)-8 and (-)-8) and 2-dipropylamino-8-hydroxytetralin (compound 20) are shown in Table 2. The resolved isomers were found to be about as potent as the corresponding racemates.

b. Castrated rats (preliminary results)

Out of 9 castrated male rats treated with 8 mg/kg of racemic compound 8, 5 mounted the female, 2 of these 5 made intromissions and 1 of these 2 ejaculated, while none out of 9 saline-treated castrated animals showed any sexual activity.

TABLE 2

| Compound | Dose (mg/kg) | N | Ejaculatory latency (min)[a] | Intromissions (number)[a] | Mounts (number)[a] | Postejaculatory interval (min)[a] |
|---|---|---|---|---|---|---|
| 8 · HCl | 1 | 12 | 3.20 | 8 | 0 *b) | 6.40 |
| 8 · HCl | 2 | 12 | 2.65 | 7 *** | 1 | 5.05 |
| 8 · HCl | 4 | 14 | 1.55 *** | 3.5 *** | 1 *** | 4.55 |
| 8 · HCl | 8 | 14 | 0.45*** | 1.5 *** | 0 ** | 5.05 |
| 8 · HCl | 16 | 14 | 0.55*** | 1 *** | 0 *** | 4.55 |
| (+)-8 · HCl | 4 | 20 | 1.05*** | 2 *** | 1 * | 6.15 |
| (-)-8 · HCl | 4 | 20 | 1.30 *** | 4.5 ** | 0.5* | 5.65 |
| Control | - | 14 | 4.75 | 9 | 2 | 5.50 |
| | | | | | | |
| 20 | 4 | 9 | 0.25 | 1 *** | 0 | 4.75 |
| Control | - | 9 | 1.80 | 5 | 1 | 4.45 |

a) Medians are given.

b) Statistics: Wilcoxon matched-pair sign rank's test (two-tailed)

Significances: * $p < 0.05$, ** $p < 0.02$, *** $p < 0.01$; All other values NS.

Conclusion

The pharmacological data affirm that the compounds of the present invention are very potent centrally acting 5-HT-receptor stimulating agents with high selectivity for 5-HT over DA and NA receptors. At present, no compound with such properties seems to be known in the literature. In addition to this, the compounds have stronger effect than any other known compound on the masculine sexual behaviour. Due to this unique pharmacological profile, the compounds are of great clinical interest in the treatment of certain disorders in the CNS such as depression and sexual disturbances. Additional potential uses of the new compounds are in the control of pain, where 5-HT has been shown to be involved, and in senile disturbances of movement and mental function e.g. senile dementia, where a decreased level of brain 5-HT has been demonstrated.

Best mode of carrying out the invention

The compound 2-(di-n-propylamino)-8-hydroxytetralin and its salts, processes for preparing said compound and methods employing said compound in therapy represent the best mode of carrying out the invention known to the inventors at present.

CLAIMS

1. A compound of the formula

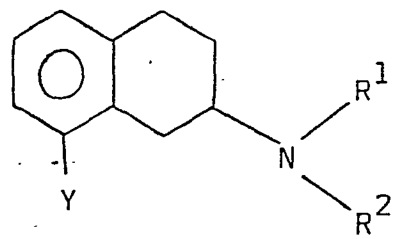

II

wherein Y is OH, $NH_2$, $R^3COO$, $R^3CONH$ or $CH_3SO_2NH$ and $R^3$ is an alkyl group having 1 to 5 carbon atoms, a benzyl or phenyl group, and $R^1$ is an alkyl group having 1 to 8 carbon atoms, a benzyl or phenethyl group and $R^2$ is hydrogen or an alkyl group having 1 to 5 carbon atoms or $R^1$ and $R^2$ together form an alkylene group having 4 to 6 carbon atoms, as the base or a pharmaceutically acceptable acid addition salt thereof.

2. A compound according to claim 1 wherein Y is OH or $R^3COO$.

3. A compound according to claim 1 or 2 wherein $R^1$ and $R^2$ are the same or different and selected from ethyl and n-propyl.

4. A compound for pharmaceutical or medical use characterized by the formula

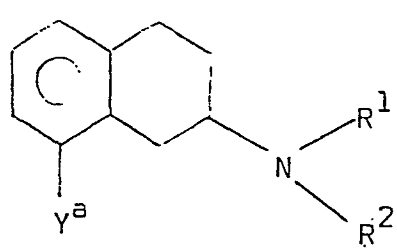

IIa

wherein $Y^a$ is $OCH_3$, OH, $NH_2$, $R^3COO$, $R^3CONH$, or $CH_3SO_2NH$ and $R^3$ is an alkyl group having 1-5 carbon atoms, a benzyl or phenyl group, and $R^1$ is an alkyl group having 1 to 8 carbon atoms, a benzyl or phenethyl group and $R^2$ is hydrogen or an alkyl group having 1 to 5 carbon atoms or $R^1$ and $R^2$ together from an alkylene group having 4 to 6 carbon atoms as the base or a pharmaceutically acceptable acid addition salt thereof.

5. A compound according to claim 4 wherein Y is $OCH_3$, OH or $R^3COO$.

6. A compound according to claim 5 wherein Y is $OCH_3$.

7. A compound according to either of claims 4 to 6 wherein $R^1$ and $R^2$ are the same or different and selected from ethyl and n-propyl.

8. A process for preparing a compound of the formula

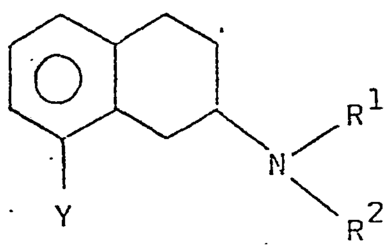

II

wherein Y is OH, $NH_2$, $R^3COO$, $R^3CONH$ or $CH_3SO_2NH$ and $R^3$ is an alkyl group having 1 to 5 carbon atoms, a benzyl or phenyl group, and $R^1$ is an alkyl group having 1 to 8 carbon atoms, a benzyl or phenethyl group and $R^2$ is hydrogen or an alkyl group having 1 to 5 carbon atoms or $R^1$ and $R^2$ together form an alkylene group having 4 to 6 carbon atoms, as the base or a pharmaceutically acceptable acid addition salt thereof, characterized in that

a) an ether of the formula

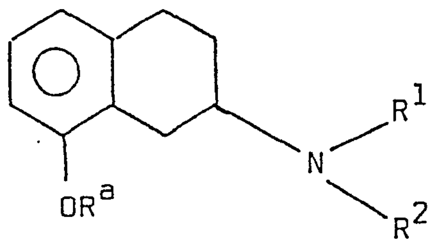

III

wherein $R^a$ represents a hydrocarbon residue, and $R^1$ and $R^2$ are as defined above, is cleaved to form a compound of formula II wherein Y is a hydroxy group,

b) a compound of formula II wherein Y is OH or $NH_2$ and $R^2$ is other than H is converted into a compound of the same formula wherein Y is $R^1COO$, $R^1CONH$ or $CH_3SO_2NH$ by treating the firstmentioned compound with an appropriate carboxylic acid halide $R^1COX$ or anhydride $(R^1CO)_2O$ or with a methylsulfonyl halide $CH_3SO_2X$ in the presence of a base,

c) a compound of formula IV

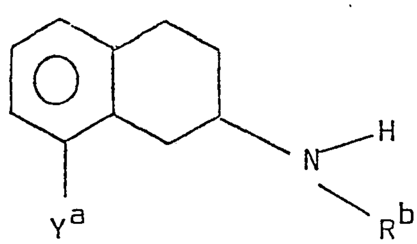

IV

wherein $R^b$ is either $R^1$ or $R^2$, and $R^1$, $R^2$ and $Y^a$ are as defined above, is converted into a compound of formula II wherein Y is OH, by alkylation of the nitrogen atom with an appropriate alkylating agent,

d) an amide of the formula

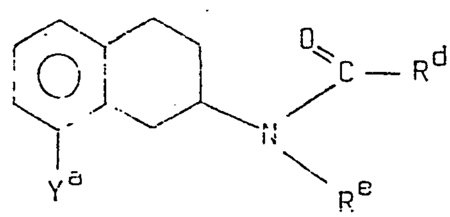

V

wherein $Y^a$ is OH or $NH_2$, $R^d$ is an alkyl group defined by the relation $R^d$-$CH_2$- equals either $R^1$ or $R^2$ and $R^e$ is the other of $R^1$ and $R^2$, is reduced to the formation of a compound of formula II,

e) a compound of the formula

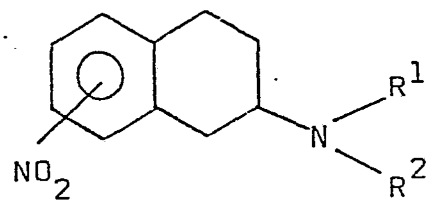

IX

is reduced to the corresponding amine, and if required an isomeric mixture as regards the position of the nitro group is chromatographically separated either before or after the reduction to the formation of the 8-amino compound of formula II, or

f) a compound of the formula

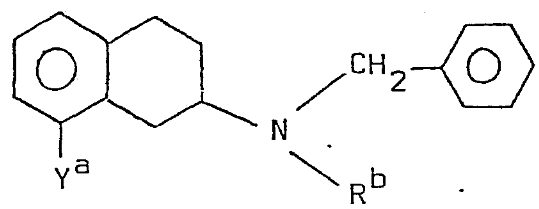

XIV

wherein $Y^a$ and $R^b$ are as defined above, is catalytically reduced to the formation of a compound of formula IV,

g) an enamine with either a $C_1$-$C_2$ or a $C_2$-$C_3$ double bond or an imine base (without $R^2$) or immonium salt (e.g. $ClO_4^-$ or $BF_4^-$) with a $C_2$-N double bond of the formula

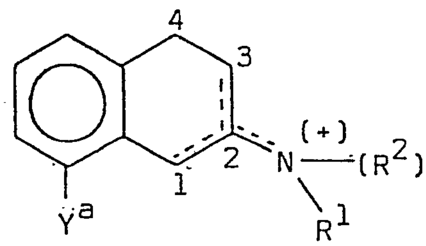

XV

wherein $Y^a$, $R^1$ and $R^2$ are as defined above, is reduced to a compound of formula II or IIa,

h) a compound of the formula

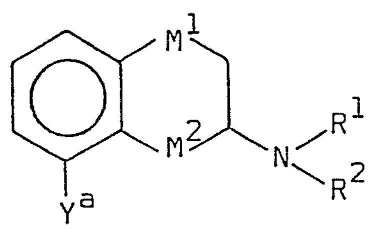

XVI

one of $M^1$ and $M^2$ represents $>CH-Z^1$ or $>C=O$ and the other represents $-CH_2-$, $>CH-Z^1$ or $>C=O$, $Z^1$ is a group sensitive to hydrogenolysis such as OH in benzylic position or halogen, and $R^1$ and $R^2$ are as defined above, is converted to a compound of formula II or IIa by reduction, or

i) a compound of the formula

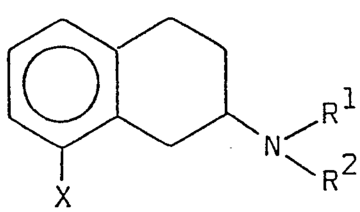

XVII

wherein X represents $SO_3H$, Cl or $NH_2$, a hydroxy group is substituted for the group X to the formation of a compound of formula II wherein Y represents a hydroxy group;

whereupon optionally a base obtained is converted to a pharmaceutically acceptable acid addition salt or a salt obtained is converted to the base or to a different, pharmaceutically acceptable acid addition salt, and optionally an isomeric mixture obtained is separated to a pure isomer.

9. A pharmaceutical preparation comprising as an active ingredient a compound according to claim 1 or claim 4, in conjunction with a pharmaceutically acceptable carrier.

10. A pharmaceutical preparation for the treatment of disorders in the central nervous system, comprising as an active ingredient a compound according to claim 1 or claim 4, in conjunction with a pharmaceutically acceptable carrier.

0041488

European Patent Office

# EUROPEAN SEARCH REPORT

Application number

EP 81 85 0092.8

| Category | DOCUMENTS CONSIDERED TO BE RELEVANT — Citation of document with indication, where appropriate, of relevant passages | Relevant to claim | CLASSIFICATION OF THE APPLICATION (Int. Cl.3) |
|---|---|---|---|
| | DE - A1 - 2 803 582 (SANDOZ-PATENT-GMBH)<br>* claim 1; page 6; fig. Ia *<br>* claim 2; page 6, last two lines *<br>* claim 3; page 22, lines 1 to 13 *<br>-- | <br>1,2,3<br>8<br>9,10 | C 07 C 91/28<br>C 07 C 93/14<br>C 07 C 87/64<br>C 07 C 85/24<br>C 07 C 85/12<br>C 07 C 89/00<br>C 07 C 101/42<br>C 07 C 101/12<br>C 07 D 295/08<br>A 61 K 31/13 |
| D | Chemical Abstracts vol. 62, no. 13, 21 June 1965<br>Columbus, Ohio USA<br>D.E. AMES et al. "The synthesis of alkoxy-1,2,3,4-tetrahydronaphthalene derivatives. I. 2-Amino-, alkylamino-, and dialkylamino derivatives"<br>abstract no. 16154b<br>& J. Chem. Soc. 1965, pages 2636 to 2641<br>-- | 7 | TECHNICAL FIELDS SEARCHED (Int. Cl.3)<br>A 61 K 31/13<br>C 07 C 87/64<br>C 07 C 91/28<br>C 07 C 93/14<br>C 07 C 101/12<br>C 07 C 101/42 |
| A<br>D,A | Chemical Abstracts, vol. 84, no. 7, 16 February 1976<br>Columbus, Ohio USA<br>D.B. RUSTERHOLZ et al. "Ergoline congeners as potential inhibitors of prolactin release 2".<br>page 12, column 1, abstract no. 38571j<br>& J. Med. Chem. 1976, vol. 19, no. 1, pages 99 to 102<br>-- | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| A | DE - A1 - 2 816 122 (BAYER AG)<br>---- | | |
| X | The present search report has been drawn up for all claims | | &: member of the same patent family, corresponding document |

| Place of search | Date of completion of the search | Examiner |
|---|---|---|
| Berlin | 08-09-1981 | PHILLIPS |

EPO Form 1503.1 06.78